# FASCICULE DE BREVET EUROPEEN

(11) **EP 2 724 794 B1**
(45) Date de publication et mention de la délivrance du brevet: **28.01.2015**
(21) Numéro de dépôt: 13187489.3
(22) Date de dépôt: 07.10.2013
(51) Int. Cl.: B09B 3/00, A61L 11/00, A61L 2/12, F26B 17/20, H05B 6/78, F26B 3/347, A62D 3/178

(54) **Système de décontamination des déchets**
Abfalldekontaminierungssystem
Waste decontamination system

(30) Priorité: 05.10.2012 FR 1259480
(43) Date de publication de la demande: 30.04.2014
(73) Titulaire: AMB, 7012 Mons (BE)
(72) Inventeur: Hurlin, Gauthier, 7012 MONS (BE)
(74) Mandataire: Cabinet Plasseraud

(56) Documents cités:
- DE-C1- 3 505 570
- FR-A1- 2 646 083
- GB-A- 2 420 542

## Description

La présente invention concerne le domaine des installations destinées à la décontamination des déchets et, plus particulièrement un système de décontamination de déchets faisant appel à un chauffage par micro-ondes.

Généralement, les systèmes de décontamination de déchets de ce type comprennent le plus souvent et principalement :
- des moyens de remplissage, de déchiquetage, de mélange et de dosage ;
- une chambre à micro-ondes avec des moyens de transport pour l'acheminement en continu des déchets le long d'au moins un émetteur de micro-ondes prévue pour élever en température les déchets ; et
- des moyens de maintien en température des déchets.

Le temps de séjour des déchets dans les moyens de maintien en température est déterminé afin de s'assurer que les déchets soient suffisamment exposés aux températures de traitement nécessaires à la décontamination des déchets.

On connait ainsi un tel système du document FR 2 646 083 qui décrit un système de décontamination de déchets répondant à cette organisation. Dans le système décrit dans ce document, les moyens de transport de la chambre à micro-ondes sont constitués par une vis sans fin animée d'un mouvement de rotation dans un conduit fermé de faible diamètre, exposé aux micro-ondes. La vis sans fin est inclinée selon un angle ascendant dans le sens de déplacement des déchets. Dans une variante de ce système, les moyens de maintien en température des déchets comprennent deux vis sans fin de transport, chacune reçue dans un conduit incliné selon un angle ascendant dans le sens de déplacement des déchets.

Toutefois, une telle solution présente un inconvénient majeur. En effet, pour certains types de déchets de grande densité, il se produit un bourrage des déchets à l'entrée du conduit des moyens de maintien en température. Ces déchets, plus lourds, s'accumulent en partie basse du berceau de la vis sans fin, et ne sont pas entrainés par la vis.

Une telle solution nécessite par ailleurs plusieurs motorisations pour actionner en rotation les vis sans fin des moyens de maintien en température, grevant le coût du système.

L'invention a notamment pour objectif de pallier les différents inconvénients de ces techniques connues.

Plus précisément, un objectif de l'invention est, au moins dans un mode de réalisation particulier, de fournir un système permettant un maintien en température des déchets simple et fiable.

L'invention a également pour objectif, au moins dans un mode de réalisation particulier, de fournir un système qui soit simple à mettre en place.

Encore un autre objectif de l'invention est, au moins dans un mode de réalisation particulier, de fournir un système qui permette un traitement sûr des déchets.

Un autre objectif de l'invention est, au moins dans un mode de réalisation, de fournir un système de faible encombrement.

Un autre objectif de l'invention est, au moins dans un mode de réalisation, de fournir un système qui facilite la manutention des déchets traités.

Ces objectifs, ainsi que d'autres qui apparaîtront plus clairement par la suite, sont atteints selon l'invention à l'aide d'un système de décontamination de déchets comportant essentiellement et successivement :
- des moyens de remplissage, de déchiquetage, de mélange et de dosage ;
- un dispositif de chauffage comprenant une entrée pour les déchets, au moins une chambre à micro-ondes avec des moyens de transport pour l'acheminement en continu des déchets le long d'au moins un émetteur de micro-ondes pour élever en température les déchets, et une sortie pour les déchets chauds ;
- des moyens de maintien en température des déchets chauds.

Selon l'invention, lesdits moyens de maintien en température des déchets chauds présentent un conduit comprenant un premier tronçon rectiligne disposé à la sortie de ladite au moins une chambre à micro-ondes, auquel succède un deuxième tronçon courbé et ledit premier tronçon et ledit deuxième tronçon desdits moyens de maintien en température des déchets chauds forment un conduit de section circulaire, ou polygonale, à l'intérieur duquel est disposée une vis sans fin déformable.

Selon un aspect avantageux de l'invention, le premier tronçon rectiligne du conduit des moyens de maintien en température des déchets chauds a un axe sensiblement horizontal.

Selon une approche particulièrement simple, le premier tronçon rectiligne du conduit des moyens de maintien en température des déchets chauds est incliné vers le bas dans le sens du transfert des déchets.

Préférentiellement, la vis sans fin s'étend de manière continue le long dudit premier tronçon rectiligne et dudit deuxième tronçon courbé.

Selon un aspect particulier de l'invention, ledit deuxième tronçon est ascendant suivant le sens de transport des déchets.

Selon l'invention, le deuxième tronçon courbé dudit conduit des moyens de maintien en température des déchets chauds est contenu dans un plan incliné par rapport à l'horizontale selon un angle α compris entre 10° et 80°, et plus préférentiellement entre 20° et 55°.

De manière avantageuse, le deuxième tronçon courbé du conduit des moyens de maintien en température des déchets chauds est suivi d'un troisième tronçon destiné à prolonger ledit deuxième tronçon de façon à repositionner l'axe de la vis sans fin horizontalement.

Selon un aspect avantageux de l'invention, ledit troisième tronçon est disposé en hauteur de façon à créer un dégagement permettant de placer un réceptacle en dessous et à l'aplomb d'une ouverture d'évacuation dudit troisième tronçon.

Selon un aspect particulier de l'invention, l'ouverture d'évacuation dudit troisième tronçon est fermée par un dispositif d'obturation, normalement fermé, et apte à s'ouvrir sous le poids des déchets traités.

Selon un mode de réalisation de l'invention, la section du conduit des moyens de maintien en température des déchets chauds est supérieure à la section offerte aux déchets dans la chambre à micro-ondes.

L'invention concerne également un système de décontamination comprenant au moins deux moyens de maintien en température des déchets montés en parallèle à la sortie du dispositif de chauffage.

D'autres caractéristiques et avantages de l'invention apparaîtront plus clairement à la lecture de la description suivante d'un mode de réalisation particulier de l'invention, donnée à titre de simple exemple illustratif et non limitatif, et des dessins annexés, parmi lesquels :
- la figure 1 est une vue en perspective du système de décontamination selon un mode de réalisation de l'invention ;
- la figure 2 est une vue schématique illustrant les plans et les axes caractéristiques des éléments principaux du système de décontamination selon la figure 1.

Comme précédemment évoqué, le principe général de l'invention repose donc sur la mise en oeuvre d'un système de décontamination de déchets comportant essentiellement et successivement :
- des moyens de remplissage 1, de déchiquetage 2, de mélange et de dosage 3;
- un dispositif de chauffage comprenant une entrée pour les déchets, au moins une chambre à micro-ondes 4 avec des moyens de transport pour l'acheminement en continu des déchets le long d'au moins un émetteur de micro-ondes pour élever en température les déchets, et une sortie pour les déchets chauds ;
- des moyens de maintien en température 10 des déchets chauds.

Dans un tel système de décontamination, les déchets sont alimentés par des moyens de remplissage 1, notamment sous la forme d'une trémie dont l'ouverture supérieure est fermée par un couvercle.

Les moyens de déchiquetage 2 peuvent comprendre un ou plusieurs broyeurs, déchiquetant par effet mécanique les déchets.

Les déchets ainsi déchiquetés sont ensuite mélangés et dosés par les moyens de dosage 3 à l'entrée dudit dispositif de chauffage qui comprend au moins une chambre à micro-ondes et notamment une chambre à micro-ondes 4. Selon un mode de réalisation particulier de l'invention, le dispositif de chauffage comprend plusieurs chambres à micro-ondes 4 disposées en parallèle et/ou en série.

Dans ce dispositif de chauffage, la température des déchets est élevée, par exemple, à une température comprise entre 70 °C et 110 °C.

Les moyens de maintien en température, à la sortie dudit dispositif de chauffage permettent de maintenir en température les déchets chauds pendant un temps nécessaire à la décontamination des déchets.

Selon l'invention, les moyens de maintien en température 10 des déchets présentent un conduit comprenant un premier tronçon 11 rectiligne disposé à la sortie du dispositif de chauffage, auquel succède un deuxième tronçon 12 courbé.

Par exemple, le système comprend lesdits moyens de remplissage 1 destinés à recevoir les déchets, suivi desdits moyens de déchiquetage 2, composés d'un broyeur équipé de lames de façon à broyer les déchets finement avant qu'ils ne soient acheminés vers un mélangeur comprenant essentiellement une cuve et une ailette de brassage par exemple, animé par un moteur électrique 6 pour homogénéiser les déchets broyés.

Les moyens de dosage 3, sont disposés en aval de moyens de mélange pour déverser une quantité de déchets broyés appropriée dans le conduit 5 des moyens d'acheminement. Les moyens de dosage 3 comprennent notamment une vis sans fin, non représentée sur les figures. L'élément de remplissage 1 peut être obturé par un couvercle.

Les déchets broyés et mélangés sont déversés à partir des moyens de dosage 3 vers le dispositif de chauffage qui comprend ladite chambre à micro-ondes 4 destinée à traiter les déchets, préalablement broyés.

Selon un mode de réalisation, la chambre à micro-ondes 4 reçoit, comme moyen de transport, une vis sans fin animée d'un mouvement de rotation dans un conduit 5 fermé de faible diamètre, exposée aux micro-ondes.

Les moyens de maintien en température 10 des déchets sont disposés à la sortie du dispositif de chauffage.

Les moyens de maintien en température 10 des déchets comprennent un premier tronçon rectiligne 11 disposé à la sortie de la chambre à micro-ondes 4, auquel succède un deuxième tronçon courbé 12, notamment de longueur supérieure à celle du premier tronçon 11. Les premier et deuxième tronçons 11 et 12 des moyens de maintien en température 10 des déchets forment un conduit de section circulaire, ou polygonale (i.e. hexagonale ou octogonale). Les tronçons 11 et 12 peuvent notamment être fabriqués dans un matériau métallique.

Selon un mode de réalisation particulier de l'invention, la température est maintenue le long des tronçons 11 et 12 au moyen de résistances électriques disposées sur la paroi extérieure desdits tronçons 11 et 12. Les résistances électriques peuvent notamment être associées à une enveloppe calorifuge externe.

Préférentiellement, la section du conduit des moyens de maintien en température 10 des déchets est supérieure à la section offerte aux déchets dans la chambre à micro-ondes 4, de manière à réduire la vitesse de transport des déchets dans le conduit des moyens de maintien en température 10 des déchets, et ainsi, assurer un temps de maintien suffisant, à la température souhaitée.

De préférence, le conduit de section circulaire, ou polygonale, formé par les premier et deuxième tronçons 11 et 12 reçoit, à l'intérieur, une vis sans fin 9 déformable sous la forme d'une spire sans âme par exemple. Cette vis sans fin 9 peut être entraînée en rotation par l'intermédiaire d'un moto-réducteur.

Selon un mode de réalisation particulier de l'invention, la vis sans fin 9 s'étend de manière continue le long du premier tronçon rectiligne 11 et du deuxième tronçon courbé 12.

La rigidité de la spire est suffisamment faible afin que la vis soit déformable pour suivre la trajectoire courbe du deuxième tronçon 12, mais assez forte pour limiter la compression de la vis, lors de son actionnement, sous l'action et la résistance des déchets.

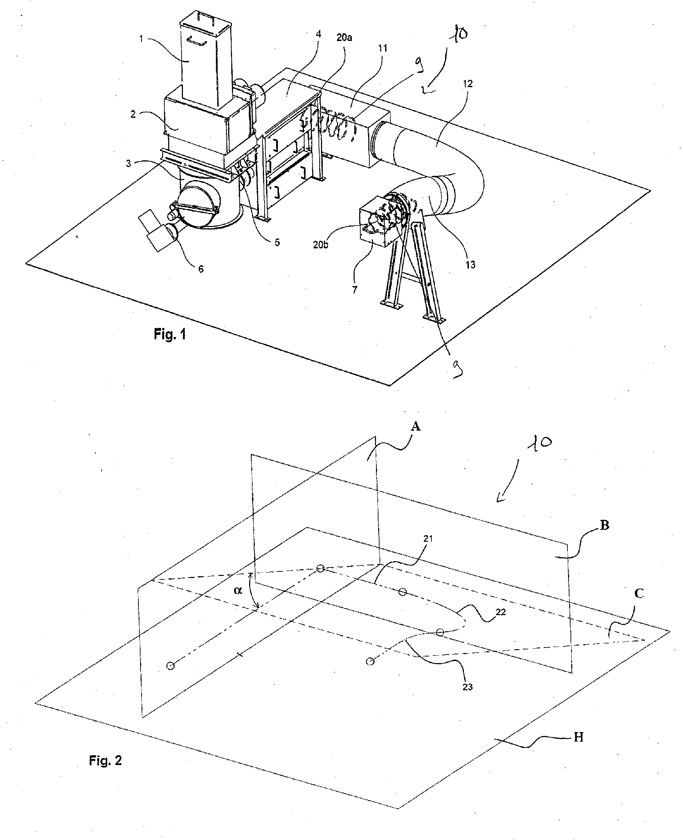

Selon ce mode de réalisation particulier de l'invention, illustré non limitativement à la figure 2, le premier tronçon 11 du conduit des moyens de maintien en température 10 des déchets a un axe sensiblement horizontal 21, parallèle à un plan H représentant le sol, et contenu dans un plan vertical B.

Selon un autre mode de réalisation particulier de l'invention, le premier tronçon rectiligne 11 du conduit des moyens de maintien en température 10 des déchets est incliné vers le bas dans le sens du transfert des déchets, de façon à favoriser l'écoulement naturel des liquides contenus dans les déchets.

De préférence, le deuxième tronçon 12 du conduit des moyens de maintien en température 10 des déchets est ascendant suivant le sens de transport des déchets. Avantageusement, l'axe courbe 22 du deuxième tronçon 12 appartient à un plan incliné C par rapport à l'horizontale, par exemple le sol, selon un angle α compris entre 10° et 80°, et plus préférentiellement entre 20° et 55°. Une telle disposition permet d'obtenir une meilleure compacité du système et évite par ailleurs le recours à un dispositif complémentaire de relevage après le maintien en température, pour le chargement de conteneurs.

Selon un mode de réalisation particulier de l'invention, le deuxième tronçon courbé 12 du conduit du système de maintien en température des déchets est suivi d'un troisième tronçon 13 destiné à prolonger le deuxième tronçon 12 de façon à repositionner l'axe de la vis sans fin 9 horizontalement selon l'axe 23.

De manière avantageuse, le troisième tronçon 13 est disposé en hauteur de façon à créer un dégagement permettant de placer un réceptacle, telle qu'une benne par exemple, en dessous et à l'aplomb d'une ouverture d'évacuation 7 disposée au niveau de la sous-face du troisième tronçon 13.

Afin de limiter les déperditions thermiques, l'ouverture d'évacuation 7 du troisième tronçon peut être fermée par un dispositif d'obturation, normalement fermé, et apte à s'ouvrir sous le poids des déchets. Un tel dispositif d'obturation peut comprendre, par exemple, une trappe mobile, normalement fermée par l'intermédiaire d'un contrepoids, ou encore sous l'action de moyens élastiques.

Selon un mode de réalisation particulier de l'invention, au moins deux moyens de maintien en température des déchets montés en parallèle peuvent être disposés à la sortie de ladite au moins une chambre à micro-ondes 4. Cette variante est particulièrement intéressante afin de réduire plus encore l'encombrement du dispositif.

Naturellement, d'autres modes de réalisation auraient pu être envisagés par l'homme du métier sans pour autant sortir du cadre de l'invention définie par les revendications ci-après.

### NOMENCLATURE

1. Moyens de remplissage,
2. Moyens de déchiquetage,
3. Moyens de mélange et de dosage,
4. Chambre à micro-ondes,
5. Conduit des moyens d'acheminement,
6. Moteur électrique,
7. Ouverture d'évacuation,
9. Vis sans fin,
10. Moyens de maintien en température des déchets chauds.
11. Premier tronçon
12. Deuxième tronçon
13. Troisième tronçon
21. Axe premier tronçon
22. Axe deuxième tronçon
23. Axe troisième tronçon

## Revendications

1. Système de décontamination de déchets comportant essentiellement et successivement :
- des moyens de remplissage (1), de déchiquetage (2), de mélange et de dosage (3) ;
- un dispositif de chauffage comprenant une entrée pour les déchets, au moins une chambre à micro-ondes (4) avec des moyens de transport pour l'acheminement en continu des déchets le long d'au moins un émetteur de micro-ondes, et une sortie pour les déchets chauds ;
- des moyens de maintien en température (10) des déchets chauds,
**caractérisé en ce que** lesdits moyens de maintien en température (10) des déchets chauds présentent un conduit comprenant un premier tronçon (11) rectiligne disposé à la sortie de ladite au moins une chambre à micro-ondes (4), auquel succède un deuxième tronçon courbé (12), ledit premier tronçon (11) et ledit deuxième tronçon (12) formant un conduit de section circulaire, ou polygonale, à l'intérieur duquel est disposée une vis sans fin (9) déformable.

2. Système de décontamination selon la revendication 1, dans lequel le premier tronçon (11) rectiligne du conduit des moyens de maintien en température (10) des déchets chauds a un axe sensiblement horizontal.

3. Système de décontamination selon la revendication 1, dans lequel le premier tronçon (11) rectiligne du conduit des moyens de maintien en température (10) des déchets chauds est incliné vers le bas dans le sens du transfert des déchets.

4. Système de décontamination selon l'une des revendications 1 à 3, dans lequel la vis sans fin (9) s'étend de manière continue le long dudit premier tronçon (11) rectiligne et dudit deuxième tronçon (12) courbé.

5. Système de décontamination selon l'une des revendications 1 à 4, dans lequel ledit deuxième tronçon (12) est ascendant suivant le sens de transport des déchets.

6. Système de décontamination selon la revendication 5, dans lequel le deuxième tronçon (12) courbé dudit conduit des moyens de maintien en température (10) des déchets chauds est contenu dans un plan incliné par rapport à l'horizontale selon un angle α compris entre 10° et 80°.

7. Système de décontamination selon la revendication 6, dans lequel le deuxième tronçon (12) courbé dudit conduit des moyens de maintien en température (10) des déchets chauds est contenu dans un plan incliné par rapport à l'horizontale selon un angle α compris entre 20° et 55°.

8. Système de décontamination selon l'une des revendications 1 à 7, dans lequel le deuxième tronçon (12) courbé du conduit des moyens de maintien en température (10) des déchets chauds est suivi d'un troisième tronçon (13) destiné à prolonger ledit deuxième tronçon (12) de façon à repositionner l'axe de la vis sans fin (9) horizontalement.

9. Système de décontamination selon à la revendication 8, dans lequel ledit troisième tronçon (13) est disposé en hauteur de façon à créer un dégagement permettant de placer un réceptacle en dessous et à l'aplomb d'une ouverture d'évacuation (7) dudit troisième tronçon (13).

10. Système de décontamination selon la revendication 9, dans lequel l'ouverture d'évacuation (7) dudit troisième tronçon (13) est fermée par un dispositif d'obturation normalement fermé et apte à s'ouvrir sous le poids des déchets traités

11. Système de décontamination selon l'une des revendications 1 à 10, dans lequel la section du conduit des moyens de maintien en température (10) des déchets chauds est supérieure à la section offerte aux déchets dans la chambre à micro-ondes (4).

12. Système de décontamination selon l'une des revendications 1 à 11 comprenant au moins deux moyens de maintien en température (10) des déchets montés en parallèle à la sortie du dispositif de chauffage.

## Patentansprüche

1. System zur Dekontamination von Abfällen, im Wesentlichen und aufeinanderfolgend umfassend:
- Mittel zum Befüllen (1), Zerkleinern (2), Mischen und Dosieren (3);
- eine Erhitzungsvorrichtung, umfassend einen Eingang für die Abfälle, mindestens eine Mikrowellenkammer (4) mit Transportmitteln für die kontinuierliche Beförderung der Abfälle entlang mindestens eines Mikrowellensenders und einen Ausgang für die heißen Abfälle;
- Mittel (10) zum Halten der heißen Abfälle auf Temperatur,
**dadurch gekennzeichnet, dass** die Mittel (10) zum Halten der Abfälle auf Temperatur eine Leitung aufweisen, umfassend einen ersten geraden Abschnitt (11), der am Ausgang der mindestens einen Mikrowellenkammer (4) angeordnet ist, auf den ein zweiter gekrümmter Abschnitt (12) folgt, wobei der erste Abschnitt (11) und der zweite Abschnitt (12) eine Leitung mit kreisförmigem oder polygonalem Querschnitt bilden, in der eine verformbare Schnecke (9) angeordnet ist.

2. System zur Dekontamination nach Anspruch 1, bei dem der erste gerade Abschnitt (11) der Leitung der Mittel (10) zum Halten der heißen Abfälle auf Temperatur eine im Wesentlichen horizontale Achse hat.

3. System zur Dekontamination nach Anspruch 1, bei dem der erste gerade Abschnitt (11) der Leitung der Mittel (10) zum Halten der heißen Abfälle auf Temperatur nach unten in Richtung der Weiterleitung der Abfälle geneigt ist.

4. System zur Dekontamination nach einem der Ansprüche 1 bis 3, bei dem sich die Schnecke (9) kontinuierlich entlang des ersten geraden Abschnitts (11) und des zweiten gekrümmten Abschnitts (12) erstreckt.

5. System zur Dekontamination nach einem der Ansprüche 1 bis 4, bei dem der zweite Abschnitt (12) in Transportrichtung der Abfälle ansteigend ist.

6. System zur Dekontamination nach Anspruch 5, bei dem der zweite gekrümmte Abschnitt (12) der Leitung der Mittel (10) zum Halten der heißen Abfälle auf Temperatur in einer zur Horizontalen in einem Winkel α zwischen 10° und 80° geneigten Ebene enthalten ist.

7. System zur Dekontamination nach Anspruch 6, bei dem der zweite gekrümmte Abschnitt (12) der Leitung der Mittel (10) zum Halten der heißen Abfälle auf Temperatur in einer zur Horizontalen in einem Winkel α zwischen 20° und 55° geneigten Ebene enthalten ist.

8. System zur Dekontamination nach einem der Ansprüche 1 bis 7, bei dem auf den zweiten gekrümmten Abschnitt (12) der Leitung der Mittel (10) zum Halten der heißen Abfälle auf Temperatur ein dritter Abschnitt (13) folgt, der dazu bestimmt ist, den zweiten Abschnitt (12) zu verlängern, um die Achse der Schnecke (9) wieder horizontal zu positionieren.

9. System zur Dekontamination nach Anspruch 8, bei dem der dritte Abschnitt (13) in der Höhe angeordnet ist, um einen Freiraum zu schaffen, der es ermöglicht, einen Behälter unter und senkrecht zu einer Entleerungsöffnung (7) des dritten Abschnitts (13) anzuordnen.

10. System zur Dekontamination nach Anspruch 9, bei dem die Entleerungsöffnung (7) des dritten Abschnitts (13) durch eine normalerweise geschlossene Verschlussvorrichtung geschlossen ist, die geeignet ist, sich unter dem Gewicht der bearbeiteten Abfälle zu öffnen.

11. System zur Dekontamination nach einem der Ansprüche 1 bis 10, bei dem der Querschnitt der Leitung der Mittel (10) zum Halten der heißen Abfälle auf Temperatur größer als der Querschnitt ist, der den Abfällen in der Mikrowellenkammer (4) geboten wird.

12. System zur Dekontamination nach einem der Ansprüche 1 bis 11, umfassend mindestens zwei Mittel (10) zum Halten der Abfälle auf Temperatur, die parallel am Ausgang der Erhitzungsvorrichtung montiert sind.

## Claims

1. Waste decontamination system comprising substantially and successively:
- a filling unit (1), a shredding unit (2), a mixing and metering unit (3);
- a heating device comprising an inlet for waste, at least one microwave chamber (4) with a transport unit for the continuous conveying of waste along at least one microwave transmitter, and an outlet for the hot waste;
- means for maintaining the temperature (10) of the hot waste,
**characterised in that** said means for maintaining the temperature (10) of the hot waste has a conduit comprising a first rectilinear section (11) arranged at the output of said at least one microwave chamber (4), to which succeeds a second curved section (12), said first section (11) and said second section (12) forming a conduit of circular or polygonal section, inside of which is arranged a worm screw (9) that can be deformed.

2. Decontamination system according to claim 1, wherein the first rectilinear section (11) of the conduit of the means for maintaining the temperature (10) of the hot waste has a substantially horizontal axis.

3. Decontamination system according to claim 1, wherein the first rectilinear section (11) of the conduit of the means for maintaining the temperature (10) of the hot waste is tilted downwards in the direction of the transfer of the waste.

4. Decontamination system according to one of claims 1 to 3, wherein the worm screw (9) extends continuously along said first rectilinear section (11) and said second curved section (12).

5. Decontamination system according to one of claims 1 to 4, wherein said second section (12) is ascendant according to the direction of transport of the waste.

6. Decontamination system according to claim 5, wherein the curved second section (12) of said means for maintaining the temperature (10) of the hot waste is contained within a plane that is tilted with respect to the horizontal according to an angle α between 10° and 80°.

7. Decontamination system according to claim 6, wherein the second curved section (12) of said conduit of the means for maintaining the temperature (10) of the hot waste is contained in a plane that is tilted with respect to the horizontal according to an angle α between 20° and 55°.

8. Decontamination system according to one of claims 1 to 7, wherein the second curved section (12) of the conduit of the means for maintaining the temperature (10) of the hot waste is followed by a third section (13) intended to prolong said second section (12) in such a way as to reposition the axis of the worm screw (9) horizontally.

9. Decontamination system according to claim 8, wherein said third section (13) is arranged at a height so as to create a clearance that makes it possible to place a container underneath and immediately under an evacuation opening (7) of said third section (13).

10. Decontamination system according to claim 9, wherein the evacuation opening (7) of said third section (13) is closed by a system for closing off that is normally closed and able to open under the weight of the processed waste.

11. Decontamination system according to one of claims 1 to 10, wherein the section of the conduit of the means for maintaining the temperature (10) of the hot waste is greater than the section offered to the waste in the microwave chamber (4).

12. Decontamination system according to one of claims 1 to 11 comprising at least two means for maintaining the temperature (10) of waste mounted in parallel at the output of the heating device.
